# EUROPEAN PATENT APPLICATION

(11) **EP 3 967 751 A1**
(43) Date of publication of application: **16.03.2022**
(21) Application number: 20781916.0
(22) Date of filing: 02.04.2020
(51) Int. Cl.: C12N 5/077

(54) **HUMAN CARDIOMYOCYTE SEPARATION REAGENT, CULTURE MEDIUM, SEPARATION METHOD, AND CULTURE METHOD**

(30) Priority: 04.04.2019 CN 201910275447
(71) Applicant: Fuwai Hospital of Chinese Academy of Medical Sciences, Beijing 100037 (CN)
(72) Inventor: HU, Shengshou, Beijing 100037 (CN); ZHOU, Bingying, Beijing 100037 (CN); HOU, Yongfeng, Beijing 100037 (CN); TANG, Xiaoli, Beijing 100037 (CN); SHI, Xun, Beijing 100037 (CN)
(74) Representative: Zwicker, Jörk
(86) International application number: PCT/CN2020/082949
(87) International publication number: WO 2020/200272

(57) **Abstract**

Provided are a composition containing (-)-Blebbistain and/or para-Blebbistain, a kit comprising the composition, and a cardiomyocyte culture method using the composition as a culture medium. Also provided is a use of (-)-Blebbistain and/or para-Blebbistain in preparation of a cardiomyocyte isolation reagent or cardiomyocyte culture reagent.

## Description

The scientific research project involved in this application is the Medicine and Health Technology Innovation Project of the Chinese Academy of Medical Sciences, with the project number of 2017-I2M-1-003.

### Technical Field

The invention relates to an isolation reagent and an isolation method for isolating mammalian cardiomyocytes, especially human cardiomyocytes. The invention also relates to a culture medium and a culture method for culturing mammalian cardiomyocytes, especially human cardiomyocytes.

### Background Art

Deaths caused by cardiovascular diseases account for not less than 40% of the total deaths due to diseases, higher than tumors and other diseases, and the cardiovascular diseases are the biggest killer of human life and health. Therefore, the prevention and treatment of cardiovascular diseases is very important to human health, and the importance of scientific research on the prevention and treatment of cardiovascular diseases is self-evident.

To study the prevention and treatment of any kind of disease, there must be a good cell model. However, unlike other somatic cells, cardiomyocytes as the research model of cardiovascular diseases have the characteristics of strong motility and high oxygen consumption. These characteristics determine that their isolation and culture processes require a large amount of energy and produce a large number of metabolites; therefore, it is extremely difficult to control the isolation and culture conditions, and it is also extremely difficult to guarantee the survival rate and purity of the cells during the isolation and culture. This has brought limitations to the research of cardiovascular diseases to a large extent.

In addition, because the cardiomyocyte model can only be obtained from biological subjects, its source is very rare and precious (especially for human cardiomyocytes), and the existing isolation and culture methods cannot guarantee the cell viability. Therefore, the supply of cardiomyocytes, especially human cardiomyocytes, is far from meeting the need of clinical research. In particular, compared with the cardiomyocytes of other mammals (such as rodents), the culture and isolation of human cardiomyocytes are very challenging tasks. There are obvious species differences between humans and other species, which are reflected in the transcriptome, proteome, epigenetic modification, electrophysiology and other aspects (non-patent documents 1-4). Therefore, the translation of data obtained from animals is very difficult. These differences make human cardiomyocytes have lower tolerance to *in vitro* culture and isolation conditions than cardiomyocytes of other species, leading to more difficult culture and isolation of human cardiomyocytes than cardiomyocytes of other species. Human tissue specimens can be used to eliminate species differences in the culture of other cells; however, tissues contain various cell types, resulting in high background noise in the experimental results. In addition, frozen tissue lack biological activity, and are therefore unsuitable to perform functional experiments. Another method for obtaining human cardiomyocytes in the prior art is directed differentiation of human embryonic stem cells or induced pluripotent stem cells. Although this method partially overcomes the above-mentioned obstacles, there are still incomplete differentiation, lack of epigenetic modifications, and other problems (non-patent documents 5 and 6), thus failing to truthfully reflect true states of the diseases, and failing to promote research on cardiovascular diseases.

In summary, although the prior art provides a variety of ways to obtain cardiomyocytes from mammals, especially humans, currently there is no way to obtain cardiomyocytes or effective mimetics thereof from mammals, especially humans, with high viability and a high purity. This has severely hindered the implement of related scientific research and results in failure to provide a research basis for clinical disease diagnosis and treatment.

As mentioned above, in the prior art, although researchers have begun to explore the isolation and culture of cardiomyocytes very early, there have been only a few reports so far on the culture of non-human mammalian cardiomyocytes (non-patent documents 7 and 8), in which 2,3-butanedione monoxime (hereinafter abbreviated as "BDM") is mainly used. In addition, there has not been any report on culture or isolation of human cardiomyocytes, especially human primary cardiomyocytes (hPCM).

BDM is a skeletal muscle myosin II ATPase inhibitor, which can inhibit the contraction of skeletal muscle and cardiomyocytes. BDM, as a chemical phosphatase, is presumed to affect cell contraction by affecting calcium ion channels, transient outward potassium channels and sodium-calcium exchange, blocking gap junction intercellular communication, and other means. Studies have shown that the addition of BDM during the isolation and culture of adult mouse and rat cardiomyocytes can remarkably increase the number of living cells and prolong the *in vitro* culture time. There have been also reports that BDM can protect cardiomyocytes by stabilizing the cell membrane structure and maintaining the stability of the cytoskeleton to increase glycolysis-produced ATPs and negatively regulate myocardial contractility. However, because the culture and isolation effect of BDM is not good enough to meet the needs of the scientific research and industry, the conception of using BDM to culture and isolate mammalian cardiomyocytes cannot be implemented yet, especially for the culture and isolation of human cardiomyocytes.

### Prior art documents

Non-patent document 1: Stephane N. Hatem and Martin Morad∗ James S. K. Sham, "Species Differences in the Activity of the Na(+)-Ca2+ Exchanger in Mammalian Cardiac Myocytes", Journal of Physiology (1995).
Non-patent document 2: K. W. LINZ∗ R. MEYER∗t, R. SURGES∗, S. MEINARDUS∗, J. VEES∗, A. HOFFMANN∗, 0. WINDHOLZ∗ AND C. GROHEt, "Rapid Modulation of L-Type Calcium Current by Acutely Applied Oestrogens in Isolated Cardiac Myocytes from Human, Guinea-Pig and Rat", Experimental Physiology (1998).
Non-patent document 3: S. Werfel, S. Nothjunge, T. Schwarzmayr, T. M. Strom, T. Meitinger, and S. Engelhardt, "Characterization of Circular Rnas in Human, Mouse and Rat Hearts", J Mol Cell Cardiol, 98 (2016), 103-7.
Non-patent document 4: Jr. William A. Clark, Richard A. Chizzonite, Alan W. Everett, Murray Rabinowitz, and Radovan Zak, "Species Correlations between Cardiac Isomyosins. A Comparison of Electrophoretic and Immunological Properties", THE JOURNAL OF BIOLOGICACL CHEMISTRY (1981).
Non-patent document 5: D. M. DeLaughter, A. G. Bick, H. Wakimoto, D. McKean, J. M. Gorham, I. S. Kathiriya, J. T. Hinson, J. Homsy, J. Gray, W. Pu, B. G. Bruneau, J. G. Seidman, and C. E. Seidman, "Single-Cell Resolution of Temporal Gene Expression During Heart Development", Dev Cell, 39 (2016), 480-90.
Non-patent document 6: X. Yang, L. Pabon, and C. E. Murry, "Engineering Adolescence: Maturation of Human Pluripotent Stem Cell-Derived Cardiomyocytes", Circ Res, 114 (2014), 511-23.
Non-patent document 7: M. Ackers-Johnson, P. Y. Li, A. P. Holmes, S. M. O'Brien, D. Pavlovic, and R. S. Foo, "A Simplified, Langendorff-Free Method for Concomitant Isolation of Viable Cardiac Myocytes and Nonmyocytes from the Adult Mouse Heart", Circ Res, 119 (2016), 909-20.
Non-patent document 8: E. J. Sharpe, J. R. St Clair, and C. Proenza, "Methods for the Isolation, Culture, and Functional Characterization of Sinoatrial Node Myocytes from Adult Mice", J Vis Exp (2016).

### Summary of the Invention

In order to solve the problem of isolation and culture of mammalian cardiomyocytes (especially human cardiomyocytes), the inventors of the present application has conducted in-depth research and continuous optimization on the isolation and culture technique of cardiomyocytes, taking into account the actual needs of clinical practice, and thus discovered the technical solutions of the present invention which can obtain the technical effects of more truly reflecting the state of cardiomyocytes and making a higher clinical translation possible.

The purpose of the present invention is to provide technical solutions that can be used to effectively isolate and culture cardiomyocytes, comprising an isolation reagent, a culture medium, and an isolation and culture method. By adopting the technical solutions of the present invention, the cells can be maintained in an optimal state and the morphology, survival rate and purity of the cells can be maintained during culture.

The inventors of the present application have conducted a large number of extensive and in-depth studies, and unexpectedly found that (-)-Blebbistatin or its derivative para-aminoblebbistatin can maintain the morphology and survival rate of cardiomyocytes well in the isolation and culture of mammalian cardiomyocytes (especially human cardiomyocytes), and play a key role in the isolation and culture of cardiomyocytes. The inventors of the present application also found that combining (-)-Blebbistatin or para-aminoblebbistatin with other components, in isolation and cultivation under certain conditions, can well solve the above-mentioned technical problems to be solved by the present invention, thus completing the present invention.

The first embodiment of the present invention relates to a composition that can be used as a cardiomyocyte isolation reagent, comprising (-)-Blebbistatin and/or para-aminoblebbistatin, and optionally one or more components selected from energy components, metabolic regulators, acidifying or alkalizing agents, antibiotics and isotonic agents, wherein,
(-)-Blebbistatin is a compound represented by the following Formula (1), 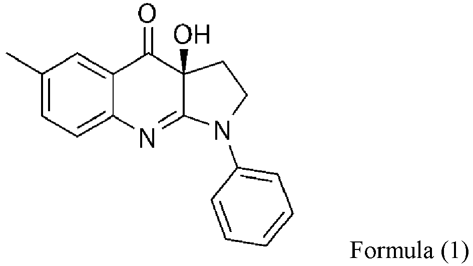
para-aminoblebbistatin is a compound represented by the following Formula (2).

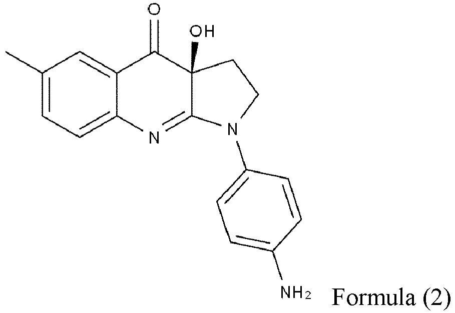

In a preferred embodiment, the composition comprises (-)-Blebbistatin and/or para-aminoblebbistatin, NaCl, KCl, MgCl₂ (such as its hexahydrate), NaH₂PO₄, 4-hydroxyethyl piperazine ethanesulfonic acid, glucose, β-aminoethanesulfonic acid, creatine, sodium pyruvate, penicillin and streptomycin.

The second embodiment of the present invention relates to a method for isolating cardiomyocytes, comprising the steps of calcium-free perfusion, digestion and cell collection, wherein the composition as described in the aforementioned first embodiment is used as a calcium-free solution in the calcium-free perfusion step.

The third embodiment of the present invention relates to a composition that can be used for the culture of cardiomyocytes, which is obtained by adding (-)-Blebbistatin and/or para-aminoblebbistatin, antibiotics and serum to a culture medium known useful for cell culture (preferably one or more culture media selected from M199 series, MEM series, and DMEM series).

The fourth embodiment of the present invention relates to a kit that can be used for the culture of cardiomyocytes, comprising:
(1) the composition described in the aforementioned third embodiment; and
(2) a coating composition, comprising Matrigel or laminin.

The fifth embodiment of the present invention relates to a method for culturing cardiomyocytes, wherein the composition described in the aforementioned third embodiment is used as a culture medium.

In this embodiment, a culture plate is coated with a coating composition comprising Matrigel or laminin, before culturing the cells.

The sixth embodiment of the present invention relates to the use of (-)-Blebbistatin and/or para-aminoblebbistatin in the preparation of a cardiomyocyte isolation reagent or a cardiomyocyte culture reagent.

By adopting the technical solutions of the present invention, human cardiomyocytes can be maintained in the optimal state, and the cell morphology, viability and purity can be maintained during the isolation and culture, thereby laying a solid foundation for cardiovascular disease research, clinical transformation, drug research and development, and individualized treatment.

### Brief Description of the Drawings

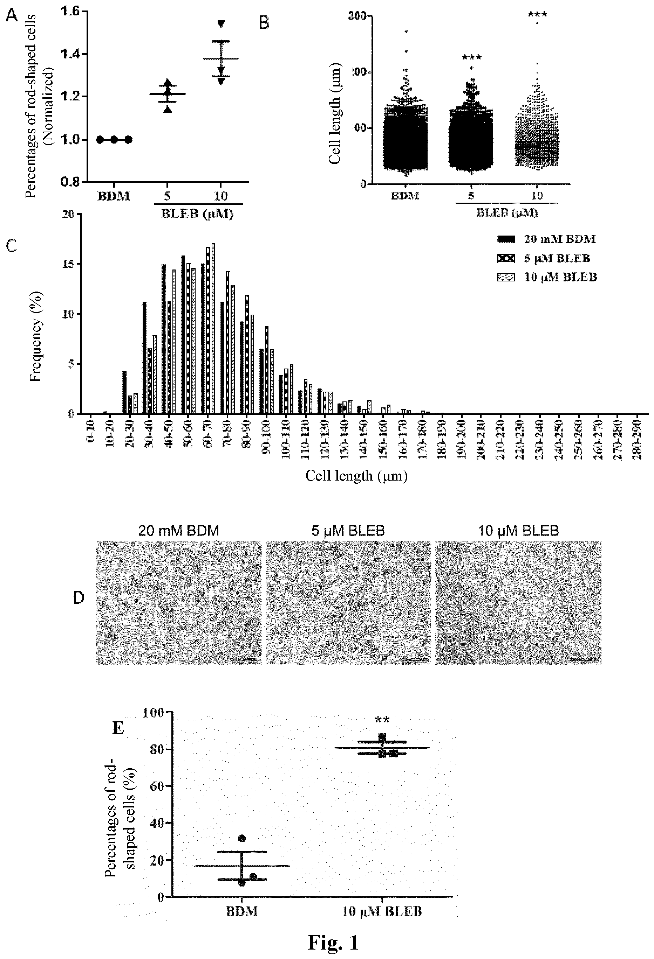

Figure 1, panel A is a graph showing the percentages of rod-shaped cardiomyocytes isolated in the 20 mM BDM group, and 5 µM and 10 µM (-)-Blebbistatin group; panels B and C are graphs showing the cardiomyocyte lengths in 20 mM BDM group, and 5 µM and 10 µM (-)-Blebbistatin group; panel D shows photomicrographs of human cardiomyocytes after isolation; and panel E is a graph showing the percentages of rod-shaped cardiomyocytes after culturing with media comprising 20 mM BDM and 10 µM (-)-Blebbistatin respectively for 5 days, after isolation of cardiomyocytes in 20 mM BDM and 10 µM (-)-Blebbistatin groups.
Figure 2, panel A is a graph showing the effect of different concentrations of (-)-Blebbistatin on the rate of rod-shaped cardiomyocytes, panel B is a graph showing the effect of different concentrations of (-)-Blebbistatin on the state of cardiomyocytes, and panel C is a graph showing the effect of different concentrations of para-aminoblebbistatin on the survival rate of cardiomyocytes.
Figure 3 is a graph showing the effect of different coating compositions on the number of adherent cardiomyocytes.
Figure 4 is a graph showing the effect of different serum concentrations on the average rate of rod-shaped cardiomyocytes.
Figure 5 is a graph showing the effect of different types of ready-prepared media on the survival rate of cardiomyocytes.
Figure 6 is a graph showing the effect of myosin inhibitors N-benzyl-p-toluenesulfonamide (BTS) and MYK-461 on the survival rate of cardiomyocytes.
Figure 7 is a graph showing the survival rate of cardiomyocytes in the (-)-Blebbistatin group and the PICCT-added group.
Figure 8 is a graph showing the result of comparison between the culture medium of the present invention and the ACCIT medium.
Figure 9, panel A is a graph showing the change in the rate of rod-shaped cardiomyocytes over the culture time; panel B is a graph showing the change in the cardiomyocyte lengths over the culture time; and panel C shows microscopic images of cardiomyocytes after different culture times.
Figure 10, panel A is a graph showing the change in the rate of rod-shaped cardiomyocytes over the culture time; panel B is a graph showing the change in the cardiomyocyte lengths over the culture time; panel C is a graph showing the change in the cardiomyocyte width over the culture time; and panel D shows microscopic images of cardiomyocytes after different culture times.
Figure 11 is a graph showing the trend of up- or down-regulation of cardiomyocyte genes.

Note: in the drawings, "BLEB" refers to "(-)-Blebbistatin", and "PAB" refers to "para-aminoblebbistatin".

### Detailed Description of Embodiments

The inventors of the present application have conducted a large number of extensive and in-depth studies, conducted a large number of experiments and explorations, and unexpectedly found that (-)-Blebbistatin or para-aminoblebbistatin can well solve the technical problems to be solved by the present invention.

(-)-Blebbistatin is a known non-muscle myosin II inhibitor and cell permeability inhibitor, and para-aminoblebbistatin is a derivative of (-)-Blebbistatin and is similar to (-)-Blebbistatin in structure and properties. In the cardiovascular physiology research, (-)-Blebbistatin is usually used as a specific decoupling agent and the like; however, it is known that (-)-Blebbistatin has some unfavorable chemical properties, for example, instability to light, phototoxicity and cytotoxicity, high fluorescence, low water solubility (its solubility is only 10.9±0.9 µM), etc. Because of these unfavorable properties, researchers have a bias against the application of (-)-Blebbistatin and its derivative para-aminoblebbistatin. So far, there has been no report about use of (-)-Blebbistatin or para-aminoblebbistatin in isolation or culture of cardiomyocytes, especially human cardiomyocytes. However, the inventors of the present application have surprisingly discovered that (-)-Blebbistatin and para-aminoblebbistatin can unexpectedly maintain the original morphology and survival rate of cardiomyocytes during the culture and isolation. On this basis, the inventors of the present application use (-)-Blebbistatin and/or para-aminoblebbistatin as the key component(s) in the culture and isolation compositions, and combine them with other specific components to perform culture and isolation, thereby solving the above-mentioned problems to be solved in the invention well and thus completing the invention.

Detailed description of the various embodiments of the present invention will be described below respectively.

The term "about" used in the context of this description means a range that fluctuates 10% above and below the cited value. For example, if the concentration of a component is about 5 mM, it means that its concentration is 4.5-5.5 mM; and if the concentration of a component is about 5-10 mM, it means that its concentration is in the range of 4.5-11 mM

The effects of some components in each composition are summarized below. It should be noted that any component may have more than one effect, and their actual effects in the overall composition may be unpredictable. For example, although sodium dihydrogen phosphate is listed as an isotonic agent in this description, it also has the function of pH adjustment and buffering, and can also be regarded as an acidifying or alkalizing agent. Similarly, although glucose is listed as an energy substance in this description, the substance also plays a role in maintaining osmotic pressure (i.e., an isotonic effect), and can also be regarded as an isotonic agent, and so on.

Unless otherwise specified, the cardiomyocytes described herein are mammalian cardiomyocytes, preferably human cardiomyocytes, such as human primary cardiomyocytes.

### (I) Compositions for isolating cardiomyocytes

The composition for isolating cardiomyocytes of the present invention comprises (-)-Blebbistatin and/or para-aminoblebbistatin. In one embodiment, the concentration of (-)-Blebbistatin in the composition is 1-50 µM, more preferably 2.5-20 µM, more preferably 3-15 µM, more preferably about 5-10 µM. In one embodiment, the concentration of para-aminoblebbistatin in the composition is 1-100 µM, more preferably 5-50 µM, more preferably 8-25 µM, and more preferably about 10-20 µM.

In one embodiment, the composition for isolation optionally further comprises one or more components selected from the group consisting of energy components, metabolic regulators, acidifying or alkalizing agents, antibiotics, and isotonic agents.

The composition for isolation optionally comprises an energy substance, which provides necessary energy reserves for the cells during the isolation. In a preferred embodiment, the energy substance is glucose with a concentration of, for example, 5-50 mM, preferably 10-30 mM, more preferably 20-25 mM, particularly preferably about 22 mM.

The composition for isolation optionally comprises a metabolic regulator, such as sodium pyruvate, insulin, creatine, β-aminoethanesulfonic acid (taurine), L-camitine, etc., which helps cells regulate the energy metabolism during the isolation. In a preferred embodiment, the metabolic regulator is sodium pyruvate, creatine and/or β-aminoethanesulfonic acid. When used alone or in combination, the concentration of sodium pyruvate may be 0.1-20 mM, preferably 1-10 mM, more preferably 3-8 mM, particularly preferably about 5 mM; the concentration of creatine may be 0.1-20 mM, preferably 1-10 mM, more preferably 3-8 mM, particularly preferably about 5 mM; and the concentration of β-aminoethanesulfonic acid may be 5-50 mM, preferably 10-30 mM, more preferably 15-25 mM, particularly preferably about 20 mM.

The composition for isolation optionally comprises an acidifying or alkalizing agent, which comprises a buffering substance for maintaining a stable pH of the composition, such as 4-hydroxyethyl piperazine ethanesulfonic acid (HEPES), and/or an acidic or basic substance for directly adjusting the pH of the composition, such as potassium hydroxide, sodium hydroxide, etc. In another preferred embodiment, the acidifying or alkalizing agent is HEPES and/or sodium hydroxide. When used alone or in combination, the concentration of HEPES is 0.5-10 mM, preferably 1-8 mM, more preferably 3-6 mM, and particularly preferably about 5 mM; and the concentration of sodium hydroxide is not specifically limited, as long as the final pH of the composition can be adjusted to 7.0-7.8, preferably 7.2-7.6, more preferably 7.3-7.5, and particularly preferably about 7.4. With reference to the above-mentioned components and their concentration ranges, those skilled in the art can select the acidifying or alkalizing agent to be used and its amount used according to specific conditions.

The composition for isolation optionally comprises an antibiotic, which functions to inhibit the growth of bacteria. The antibiotic includes, but is not limited to, penicillin, streptomycin, and Primocin. When used alone or in combination, the concentration of penicillin may be 10-500 U/ml, preferably 20-400 U/ml, more preferably 50-300 U/ml, particularly preferably about 100-200 U/ml, about 100 U/ml or about 200 U/ml; the concentration of streptomycin may be 10-500 µg/ml, preferably 20-400 µg/ml, more preferably 50-300 µg/ml, particularly preferably about 100-200 µg/ml, about 100 µg/ml or about 200 µg/ml; the concentration of Primocin can be 10-500 µg/ml, preferably 20-400 µg/ml, more preferably 50-300 µg/ml, particularly preferably about 100-200 µg/ml, about 100 µg/ml or about 200 µg/ml.

The composition for isolation optionally comprises an isotonic agent, which can maintain the osmotic pressure to ensure the water and electrolytes balance in the cells. The isotonic agent includes, but is not limited to, magnesium chloride (for example, its hydrate, such as hexahydrate), potassium chloride, sodium chloride, sodium dihydrogen phosphate, or a combination thereof. When used alone or in combination, the concentration of magnesium chloride is 0.1-20 mM, preferably 1-10 mM, more preferably 3-8 mM, particularly preferably about 5 mM; the concentration of potassium chloride is 0.1-20 mM, preferably 1-10 mM, more preferably 3-8 mM, particularly preferably about 4-5 mM; the concentration of sodium chloride is 10-500 mM, preferably about 50-200 mM, more preferably about 100-150 mM, particularly preferably about 120-130 mM; and the concentration of sodium dihydrogen phosphate is 0.5-30 mM, preferably 1-20 mM, more preferably 3-15 mM, particularly preferably about 5-10 mM

In a preferred embodiment, the composition for isolation comprises the following components: 1-50 µM (-)-Blebbistatin, 5-50 mM glucose, 0.1-20 mM sodium pyruvate, 0.1-20 mM creatine, 5-50 mM β-aminoethanesulfonic acid, 0.5-10 mM 4-hydroxyethyl piperazine ethanesulfonic acid (HEPES), 10-500 U/ml penicillin, 10-500 µg/ml streptomycin, 0.1-20 mM magnesium chloride, 0.1-20 mM potassium chloride, 10-500 mM sodium chloride, 0.5-30 mM sodium dihydrogen phosphate, and an appropriate amount of NaOH to make the pH of the composition be 7.0-7.8 (hereinafter referred to as "isolation composition 1").

In a more preferred embodiment, the composition for isolation comprises the following components: 2.5-20 µM (-)-Blebbistatin, 10-30 mM glucose, 1-10 mM sodium pyruvate, 1-10 mM creatine, 10-30 mM β-aminoethanesulfonic acid, 1-8 mM 4-hydroxyethyl piperazine ethanesulfonic acid (HEPES), 20-400 U/ml penicillin, 20-400 µg/ml streptomycin, 1-10 mM magnesium chloride, 1-10 mM potassium chloride, 50-200 mM sodium chloride, 1-20 mM sodium dihydrogen phosphate, and an appropriate amount of NaOH to make the pH of the composition be 7.2-7.6 (hereinafter referred to as "isolation composition 2").

In a particularly preferred embodiment, the composition for isolation comprises the following components: 3-15 µM (-)-Blebbistatin, 20-25 mM glucose, 3-8 mM sodium pyruvate, 3-8 mM creatine, 15-25 mM β-aminoethanesulfonic acid, 3-6 mM 4-hydroxyethyl piperazine ethanesulfonic acid (HEPES), 50-300 U/ml penicillin, 50-300 µg/ml streptomycin, 3-8 mM magnesium chloride, 3-8 mM potassium chloride, 100-150 mM sodium chloride, 3-15 mM sodium dihydrogen phosphate, and an appropriate amount of NaOH to make the pH of the composition be 7.3-7.5 (hereinafter referred to as "isolation composition 3").

In a preferred embodiment, the composition for isolation comprises the following components: 1-100 µM para-aminoblebbistatin, 5-50 mM glucose, 0.1-20 mM sodium pyruvate, 0.1-20 mM creatine, 5-50 mM β-aminoethanesulfonic acid, 0.5-10 mM 4-hydroxyethyl piperazine ethanesulfonic acid (HEPES), 10-500 U/ml penicillin, 10-500 µg/ml streptomycin, 0.1-20 mM magnesium chloride, 0.1-20 mM potassium chloride, 10-500 mM sodium chloride, 0.5-30 mM sodium dihydrogen phosphate, and an appropriate amount of NaOH to make the pH of the composition be 7.0-7.8 (hereinafter referred to as "isolation composition 4").

In a more preferred embodiment, the composition for isolation comprises the following components: 5-50 µM para-aminoblebbistatin, 10-30 mM glucose, 1-10 mM sodium pyruvate, 1-10 mM creatine, 10-30 mM β-aminoethanesulfonic acid, 1-8 mM 4-hydroxyethyl piperazine ethanesulfonic acid (HEPES), 20-400 U/ml penicillin, 20-400 µg/ml streptomycin, 1-10 mM magnesium chloride, 1-10 mM potassium chloride, 50-200 mM sodium chloride, 1-20 mM sodium dihydrogen phosphate, and an appropriate amount of NaOH to make the pH of the composition be 7.2-7.6 (hereinafter referred to as "isolation composition 5").

In a particularly preferred embodiment, the composition for isolation comprises the following components: 8-25 µM para-aminoblebbistatin, 20-25 mM glucose, 3-8 mM sodium pyruvate, 3-8 mM creatine, 15-25 mM β-aminoethanesulfonic acid, 3-6 mM 4-hydroxyethyl piperazine ethanesulfonic acid (HEPES), 50-300 U/ml penicillin, 50-300 µg/ml streptomycin, 3-8 mM magnesium chloride, 3-8 mM potassium chloride, 100-150 mM sodium chloride, 3-15 mM sodium dihydrogen phosphate, and an appropriate amount of NaOH to make the pH of the composition be 7.3-7.5 (hereinafter referred to as "isolation composition 6").

### (II) Methods for isolating cardiomyocytes

The method for isolating cardiomyocytes comprises the steps of calcium-free perfusion, digestion and cell collection. In the calcium-free perfusion step, any of the compositions for isolating cardiomyocytes described in the present invention can be used as a calcium-free solution; in the digestion step, an enzyme solution commonly known useful for cell digestion can be added to the calcium-free solution to obtain a composition for digestion, and a serum is added to the calcium-free solution to obtain a composition for cell transport. Except for these, the remaining operations, reagents and conditions are the same as those used in the general cell isolation method in the prior art.

In one embodiment, the composition used as a digestion solution in the digestion step is obtained by adding an enzyme solution and calcium chloride to the calcium-free solution (that is, any of the compositions for isolating cardiomyocytes according to the present invention), wherein the enzyme solution is, for example, protease (such as protease XXIV) and/or collagenase (such as collagenase I and collagenase II). In one embodiment, when used alone or in combination, the concentration of the protease (such as protease XXIV) in the digestion solution is 0.1-5 U/ml, preferably 0.5-3 U/ml, more preferably 1-1.5 U/ml, particularly preferably about 1.2 U/ml; the concentration of collagenase (such as collagenase I and collagenase II) is, for example, 50-800 U/ml, preferably 100-600 U/ml, more preferably 200-400 U/ml, particularly preferably about 300 U/ml. In one embodiment, the concentration of calcium chloride in the digestion solution is 0.002-0.1 mM, preferably 0.005-0.05 mM, more preferably 0.01-0.02 mM, particularly preferably about 0.015 mM

In a preferred embodiment, the composition (with pH of 7.0-7.8) used as the digestion solution comprises the following components: 50-800 U/ml collagenase II and 0.002-0.1 mM calcium chloride, in addition to the components described for the aforementioned isolation composition 1 or 4; and in another specific embodiment, the composition additionally comprises 0.1-5 U/ml protease XXIV.

In a more preferred embodiment, the composition (with pH of 7.2-7.6) used as the digestion solution comprises the following components: 100-600 U/ml collagenase II and 0.005-0.05 mM calcium chloride, in addition to the components described for the aforementioned isolation composition 2 or 5; and in another specific embodiment, the composition additionally comprises 0.5-3 U/ml protease XXIV.

In a particularly preferred embodiment, the composition (with pH of 7.3-7.5) used as the digestion solution comprises the following components: 200-400 U/ml collagenase II and 0.01-0.02 mM calcium chloride, in addition to the components described for the aforementioned isolation composition 3 or 6; and in another specific embodiment, the composition additionally comprises 1-1.5 U/ml protease XXIV.

The digestion step of the present invention can be carried out in stages, and the same or different compositions described in the above-mentioned embodiments can be used as the digestion solution in each stage.

In another embodiment, the composition used as a cell transport solution in the cell transport step is obtained by adding a serum to the calcium-free solution (that is, any of the compositions for isolating cardiomyocytes according to the present invention).

The term "serum" herein can refer to any serum commonly used in the field of cell culture, or its main components (for example, serum albumin, etc.). In one embodiment, the serum is fetal bovine serum (FBS). In another embodiment, the serum is bovine serum albumin (BSA). In a preferred embodiment, the serum is fetal bovine serum, and the volume fraction of fetal bovine serum in the composition used as the cell transport solution is 1%-20%, preferably 2%-15%, more preferably 3%-12%, particularly preferably about 5%-10%; and/or, the serum is bovine serum albumin, and the concentration of bovine serum albumin in the composition used as the cell transport solution is 0.1-10 g/ml, preferably 0.2-5 g/ml, more preferably 0.3-1 g/ml, particularly preferably about 0.5 g/ml.

In a preferred embodiment, the composition (with a pH of 7.0-7.8) used as the cell transport solution is obtained by adding fetal bovine serum and/or bovine serum albumin to the aforementioned isolation composition 1 or 4, wherein the volume fraction of fetal bovine serum in the cardiomyocyte culture composition is 1%-20%, and the concentration of bovine serum albumin in the cardiomyocyte culture composition is 0.1-10 g/ml.

In a more preferred embodiment, the composition (with a pH of 7.2-7.6) used as the cell transport solution is obtained by adding fetal bovine serum and/or bovine serum albumin to the aforementioned isolation composition 2 or 5, wherein the volume fraction of fetal bovine serum in the cardiomyocyte culture composition is 2%-15%, and the concentration of bovine serum albumin in the cardiomyocyte culture composition is 0.2-5 g/ml.

In a particularly preferred embodiment, the composition (with a pH of 7.3-7.5) used as the cell transport solution is obtained by adding fetal bovine serum and/or bovine serum albumin to the aforementioned isolation composition 3 or 6, wherein the volume fraction of fetal bovine serum in the cardiomyocyte culture composition is 3%-12%, and the concentration of bovine serum albumin in the cardiomyocyte culture composition is 0.3-1 g/ml.

### (III) Compositions for culturing cardiomyocytes

A composition for culturing cardiomyocytes of the present invention is obtained by adding (-)-Blebbistatin and/or para-aminoblebbistatin, a serum and an antibiotic to a culture medium conventionally used for cell culture in the art. The culture medium may be, for example, one or more culture media selected from M199 series, MEM series, and DMEM series; preferably, the culture medium can be selected from M199 medium, M199 glutamine-free medium, M199-4-hydroxyethyl piperazine ethanesulfonic acid glutamine-free medium, MEM medium, MEM-GlutaMAX medium, MEM-4-hydroxyethyl piperazine ethanesulfonic acid-GlutaMAX medium, DMEM-high glucose medium, and DMEM-low glucose medium; more preferably, the culture medium can be M199 medium, MEM-GlutaMAX medium or MEM-4-hydroxyethyl piperazine ethanesulfonic acid-GlutaMAX medium . In addition, the contents of (-)-Blebbistatin and/or para-aminoblebbistatin, and the types and contents of sera and antibiotics are the same as those defined in section (I) for "Compositions for isolating cardiomyocytes" and in section (II) for "Methods for isolating cardiomyocytes" above.

In a preferred embodiment, the composition for culturing cardiomyocytes is obtained by adding the following components to any series or any one of the above-mentioned culture media (such as M199, MEM-GlutaMAX or MEM-4-hydroxyethyl piperazine ethanesulfonic acid-GlutaMAX medium) (hereinafter collectively referred to as "culture composition 1"):
- (-)-Blebbistatin and/or para-aminoblebbistatin, wherein in the composition, the concentration of (-)-Blebbistatin is 1-50 µM, and the concentration of para-aminoblebbistatin is 1-100 µM;
- penicillin, and/or streptomycin, and/or Primocin, wherein the concentration of penicillin in the composition is 10-500 U/ml, the concentration of streptomycin in the composition is 10-500 µg/ml, and the concentration of Primocin in the composition is 10-500 µg/ml; and
- fetal bovine serum and/or bovine serum albumin, wherein the volume fraction of the fetal bovine serum in the composition is 1%-20%, and the concentration of the bovine serum albumin in the composition is 0.1-10 g/ml.

In a more preferred embodiment, the composition for culturing cardiomyocytes is obtained by adding the following components to any series or any one of the above-mentioned culture media (such as M199, MEM-GlutaMAX or MEM-4-hydroxyethyl piperazine ethanesulfonic acid-GlutaMAX medium) (hereinafter collectively referred to as "culture composition 2"):
- (-)-Blebbistatin and/or para-aminoblebbistatin, wherein in the composition, the concentration of (-)-Blebbistatin is 2.5-20 µM, and the concentration of para-aminoblebbistatin is 5-50 µM;
- penicillin, and/or streptomycin, and/or Primocin, wherein the concentration of penicillin in the composition is 20-400 U/ml, the concentration of streptomycin in the composition is 20-400 µg/ml, and the concentration of Primocin in the composition is 20-400 µg/ml; and
- fetal bovine serum and/or bovine serum albumin, wherein the volume fraction of the fetal bovine serum in the composition is 2%-15%, and the concentration of the bovine serum albumin in the composition is 0.2-5 g/ml.

In a particularly preferred embodiment, the composition for culturing cardiomyocytes is obtained by adding the following components to any series or any one of the above-mentioned culture media (such as M199, MEM-GlutaMAX or MEM-4-hydroxyethyl piperazine ethanesulfonic acid-GlutaMAX medium) (hereinafter collectively referred to as "culture composition 3"):
- (-)-Blebbistatin and/or para-aminoblebbistatin, wherein in the composition, the concentration of (-)-Blebbistatin is 3-15 µM (for example, about 10 µM), and the concentration of para-aminoblebbistatin is 8-25 µM (for example, about 10 µM or 20 µM);
- Penicillin, and/or streptomycin, and/or Primocin, wherein the concentration of penicillin in the composition is 50-300 U/ml (for example, about 100 U g/ml or 200 U/ml), the concentration of streptomycin in the composition is 50-300 µg/ml (for example, about 100 µg/ml or 200 µg/ml), and the concentration of Primocin in the composition is 50-300 µg/ml (for example, about 100 µg/ml or 200 µg/ml); and
- fetal bovine serum and/or bovine serum albumin, wherein the volume fraction of fetal bovine serum in the composition is 5%-10% (for example, about 5%), and the concentration of bovine serum albumin in the composition is 0.3 -1 g/ml (for example, about 0.5 g/ml).

### (IV) Kits for culturing cardiomyocytes

The kit for culturing cardiomyocytes of the present invention comprises:
(1) any of the aforementioned compositions for culturing cardiomyocytes; and
(2) a coating composition comprising Matrigel and/or laminin, wherein the effect of Matrigel and/or laminin is to promote the growth of cells adhering to the wall. For example, when laminin is used, its concentration is 10-500 µg/ml, preferably 20-300 µg/ml, more preferably 50-200 µg/ml, particularly preferably about 50 µg/ml or about 200 µg/ml.

In a preferred embodiment, the kit comprises:
(1) any of the aforementioned culture composition 1; and
(2) coating composition 1 comprising Matrigel and/or 10-500 µg/ml laminin.

In a more preferred embodiment, the kit comprises:
(1) any of the aforementioned culture composition 2; and
(2) coating composition 2 comprising Matrigel and/or 20-300 µg/ml laminin.

In a particularly preferred embodiment, the kit comprises:
(1) any of the aforementioned culture composition 3; and
(2) coating composition 3 comprising Matrigel and/or 50-200 µg/ml laminin.

In addition, when the coating composition comprises Matrigel, the amount of Matrigel needs to be adapted to the actual volume of the well, for example, it can be 150-200 µl/well.

### (V) Methods for culturing cardiomyocytes

In the method for culturing cardiomyocytes of the present invention, the cells are cultured using the composition described in section (III) or the kit described in section (IV) above.

More preferably, in the cell culture method of the present invention, a culture plate is coated with the coating composition described in section (IV) above, and then the composition described in section (III) above is used as the culture medium to culture the cells.

### (VI) Use for preparing cardiomyocyte isolation reagents or cardiomyocyte culture reagents

The present invention also relates to the use of (-)-Blebbistatin and/or para-aminobleb bistatin in the preparation of a cardiomyocyte isolation reagent or a cardiomyocyte culture reagent, and the use mode is as described in sections (I) to (V) above.

More specific embodiments of the present invention will be illustrated by the following examples, but it should be understood that these examples are not intended to limit the scope of the present invention.

### Examples

### Example 1: Human cardiomyocyte isolation test

The cardiomyocyte isolation process generally comprises tissue transport and sectioning, calcium-free perfusion, enzyme solution digestion and cell collection. In the process, the calcium-free Tyrode's solution (calcium-free solution) was the main reagent used in the isolation process, for calcium-free perfusion, enzyme solution digestion and cell collection. The test was conducted in this invention by reference to the cardiomyocyte isolation experimental protocol described in Liang Chen, Guang-ran Guo, Man Rao, Kai Chen, Jiang-ping Song and Sheng-shou Hu, "A Modified Method for Isolation of Human Cardiomyocytes to Model Cardiac Diseases.", Journal of Translational Medicine (2018). Specific steps were as follows:
An excised left atrial appendage was immediately placed in an organ preservation solution (manufactured by the University of Wisconsin, USA) (about 4°C), and quickly transferred it to a 60 mm cell culture dish in a super clean bench, an organ preservation solution (about 4°C) was added, and the culture dish was placed on an ice box pre-cooled in a refrigerator at -20°C. Scissors were used to remove excess fat and trim it, the tissue was adhered to a specimen stage, and a microtome (Leica VT1200S, Germany) was used to slice the tissue (300 µm) which was then manually cut into pieces in the super clean bench. The cut atrial appendage pieces were divided into three groups, and the tissues were decalcified three times using the calcium-free solution of the present invention. 15-20 ml calcium-free solution was added to an Erlenmeyer flask for calcium-free perfusion for three times. The Erlenmeyer flask was shaken at room temperature, the calcium-free solution was replaced with fresh one after 2 min and 3 min respectively, and the third calcium-free perfusion time is 4 min. After finishing the calcium-free perfusion, the calcium-free solution was discarded and enzyme solution 1 (preheated to 37°C) formulated from the corresponding calcium-free solution was added respectively. After the bottle opening was wrapped with a tin foil, the flask was shaken in a 37°C water bath shaker (Bibby Stuart SBS40, UK), and the change in the color of the enzyme solution was observed at any time which gradually changed from clear to turbid. The enzyme solution was collected into a 15 ml centrifuge tube, fresh enzyme solution 1 was added to the Erlenmeyer flask, and digestion was still performed in the water bath kettle. The collected enzyme solution was placed in a pre-cooled 4°C centrifuge (Eppendorf centrifuge 5804R, Germany) for centrifugation (100×g, 1 min), and the supernatant was discarded, followed by observing whether there were cardiomyocytes in the precipitate at the tube bottom, and determining the proportion of rod-shaped cells. The second digestion is the same as the first digestion. When the rate of rod-shaped cardiomyocytes contained in the precipitate is 70% or more, the enzyme solution 1 was replaced with enzyme solution 2 formulated from the calcium-free solution used in the enzyme solution 1, and digestion was continued. This time, cardiomyocytes in the turbid enzyme solution were collected by centrifugation (4°C, 100×g, 1 min) and then resuspending cells in a 3 ml transport solution.

In the process, the calcium-free solution was a calcium-free solution comprising 5 µM (-)-Blebbistatin, 10 µM (-)-Blebbistatin, and 20 mM BDM, respectively. The compositions of the calcium-free solution and enzyme solutions 1 and 2 are shown in Tables 1-3 below.

**[Table 1]**

| Component | BDM group (20 mM) | (-)-Blebbistatin group (5 µM) | (-)-Blebbistatin group (10 µM) |
|---|---|---|---|
| NaCl (mmol/L) | 126 | 126 | 126 |
| KCl (mmol/L) | 4.4 | 4.4 | 4.4 |
| MgCl₂·6H₂O (mmol/L) | 5 | 5 | 5 |
| NaH₂PO₄ (mmol/L) | 5 | 5 | 5 |
| 4-hydroxyethyl piperazine ethanesulfonic acid (mmol/L) | 5 | 5 | 5 |
| Glucose (mmol/L) | 22 | 22 | 22 |
| β-aminoethanesulfonic acid (mmol/L) | 20 | 20 | 20 |
| Creatine (mmol/L) | 5 | 5 | 5 |
| Sodium pyruvate (mmol/L) | 5 | 5 | 5 |
| BDM (mmol/L) | 20 | - | - |
| (-)-Blebbistatin (µmol/L) | - | 5 | 10 |
| Penicillin | 100 U/ml | 100 U/ml | 100 U/ml |
| Streptomycin | 100 µg/ml | 100 µg/ml | 100 µg/ml |

| | | | |
|---|---|---|---|
| NaOH adjusts the pH value, pH=7.4 | | | |

**[Table 2]**

| Component | Concentration |
|---|---|
| Protease XXIV | 1.2 U/ml |
| Collagenase II | 300 U/ml |
| CaCl₂ | 0.015 mM |

| | |
|---|---|
| Solvent: calcium-free Tyrode's solution | |

**[Table 3]**

| Component | Concentration |
|---|---|
| Collagenase II | 300 U/ml |
| CaCl₂ | 0.015 mM |

| | |
|---|---|
| Solvent: calcium-free Tyrode's solution | |

The results were shown in Figure 1. It can be seen from Figure 1A that the rod-shaped rate of the cells isolated in the 20 mM BDM group was normalized to 1.0, and the normalized rod-shaped cell rate in the 5 µM and 10 µM (-)-Blebbistatin groups reached 1.2-1.5 (p < 0.05), which indicated that the survival rate of human cardiomyocytes was significantly improved. It can be seen from Figure 1B and Figure 1C that the lengths of human cardiomyocytes isolated in the 5 µM and 10 µM (-)-Blebbistatin groups were also remarkably longer than those in the 20 mM BDM group (p < 0.05). Figure ID shows photomicrographs (Leica, DMI4000B, 10X) of human cardiomyocytes after isolation. It can be seen that the rod-shaped cell morphology in the 20 mM BDM group was not well maintained, and a large number of spherical cells can be seen in the field of view; while both 5 µM and 10 µM (-)-Blebbistatin groups can maintain the long rod-shaped morphology of human cardiomyocytes, and the 10 µM (-)-Blebbistatin group had a better effect. The above results indicated that the group comprising isolation reagents of 5-10 µM Blebbistatin can maintain the morphology of human cardiomyocytes well and increase the rate of rod-shaped cells during cell isolation.

In addition, in order to observe whether the cell states were different when the human cardiomyocytes isolated using two calcium-free solutions (20 mM BDM and 10 µM (-)-Blebbistatin) were cultured *in vitro,* we performed recalcification on the isolated human cardiomyocytes. Specifically, it was difficult for human cardiomyocytes to survive in an environment without calcium ions, and the present invention adopted a 5-step gradient recalcification method to restore the calcium ion concentration of human cardiomyocytes. The concentration gradients of CaCl₂ (with a stock concentration of 30 mM) added to the cell collection solution were 0.08 mM (5 min), 0.25 mM (5 min), 0.5 mM (5 min), 1 mM (5 min), and 1.8 mM (15 min) in order. The human cardiomyocytes isolated in the 20 mM BDM group were cultured using M199 medium with addition of 10% fetal bovine serum (FBS) (note: the concentration percentage of fetal bovine serum in the examples and corresponding figures was volume percentage), 100 U/ml penicillin, 100 µg/ml streptomycin and 20 mM BDM. The human cardiomyocytes isolated in the 10 µM (-)-Blebbistatin group were cultured using M199 medium with addition of 10% fetal bovine serum, 100 U/ml penicillin, 100 µg/ml streptomycin, and 10 µM (-)-Blebbistatin. Specifically, the cells were placed in a 37°C incubator (with 5% CO₂, and a relative saturated humidity of 95%, Thermo Scientific Heracell VIOS CO₂ 160i, USA) and cultured for 6 days respectively.

The compositions of the cell collection solutions (Tables 1-3) are shown in Table 4 below.

The results were shown in Figure IE. The rate of rod-shaped cardiomyocytes in the 10 µM (-)-Blebbistatin group was 80% or more, which was remarkably higher than that in the 20 mM BDM group (p < 0.05). This indicated that (-)-Blebbistatin not only protected the cells in the isolation of human cardiomyocytes, but also facilitated the *in vitro* culture of human cardiomyocytes.

**[Table 4]**

| Component | Volume |
|---|---|
| Calcium-free Tyrode's solution | 36 ml |
| Fetal bovine serum | 4 ml |

### Example 2: Human cardiomyocyte culture test

### Example 2.1: The effect of different concentrations of (-)-Blebbistatin and para-aminoblebbistatin on the rate of human rod-shaped cardiomyocytes

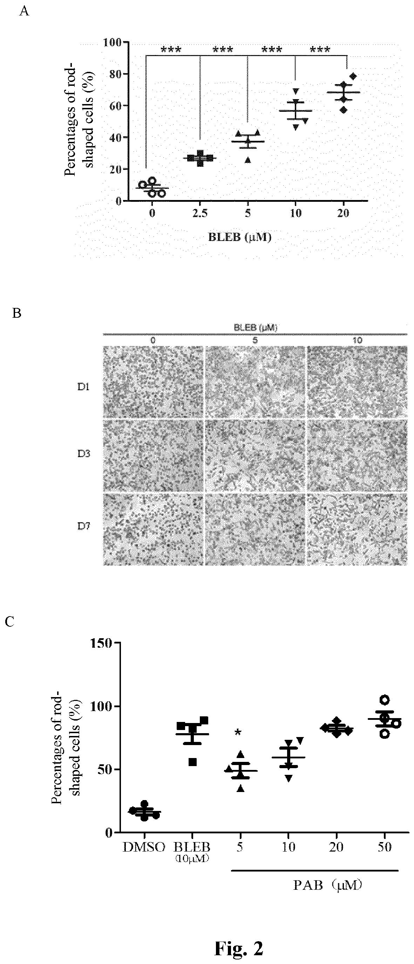

10% fetal bovine serum, 100 U/ml penicillin and 100 µg/ml streptomycin were added to M199 medium (purchased from Sigma), and the resulting medium was used as the basal medium. The experiment was divided into 9 groups, i.e. 0, 2.5, 5, 10, or 20 µM (-)-Blebbistatin, or 5 µM, 10 µM, 20 µM, or 50 µM para-aminoblebbistatin was added to the basal medium. The isolated human cardiomyocytes were divided into 9 groups, and the cells using 9 culture media were plated on a 48-well cell culture plate precoated with 200 µg laminin, and placed and cultured in a 37°C cell incubator (with 5% CO₂, and a relative saturated humidity of 95%) for 7 days, and the rates of rod-shaped cardiomyocytes were calculated. The results were shown in Figure 2.

It can be seen from Figure 2A that the concentration of (-)-Blebbistatin used was significantly related to the rate of rod-shaped cells, and the rate of rod-shaped cells in each group with addition of (-)-Blebbistatin was remarkably higher than that in the group without (-)-Blebbistatin, with a statistical difference (p < 0.001). It can be confirmed that 2.5-20 µM (-)-Blebbistatin can significantly improve the survival rate of cardiomyocytes. In addition, the rate of rod-shaped cardiomyocytes in the (-)-Blebbistatin group increased with the increase in concentration.

From the cardiomyocyte culture images shown in Figure 2B, it can be seen that the state of cardiomyocytes in the 5 µM and 10 µM (-)-Blebbistatin groups was remarkably better than in the 0 µM group after 1 day of culture. At the same time, we found that compared with the 5 µM (-)-Blebbistatin group, the 10 µM (-)-Blebbistatin group better maintained the long rod-shaped morphology of cardiomyocytes. The results were more obvious after 7 days of culture. The cell edges in the 10 µM (-)-Blebbistatin group also gradually became rounded, and the cell morphology and the number of rod-shaped cells were remarkably better than those in the 0 µM and 5 µM (-)-Blebbistatin groups. Considering the effect of (-)-Blebbistatin on cell viability and its water solubility and photosensitivity together, it can be determined that 10 µM was a more appropriate concentration of (-)-Blebbistatin for culturing cardiomyocytes.

In addition, it can be seen from Figure 2C that there was no statistical difference between the 10 µM para-aminoblebbistatin group and the 10 µM (-)-Blebbistatin group, that is, the effects of the two group on the viability of human cardiomyocytes were roughly the same. Moreover, it can be confirmed that 5-50 µM para-aminoblebbistatin (abbreviated as PAB) can significantly improve the survival rate of cardiomyocytes.

### Example 2.2: The effect of the coating composition on the number of adherent human cardiomyocytes

48-well culture plates were coated with the following reagents: laminin (Gibco, product number: 23017015; 50µg/ml or 200µg/ml, incubated overnight in a 37°C incubator, and air dried), fibronectin (Sigma Aldrich, product number: F0895-2MG; 5µg/ml or 50µg/ml, air dried), poly-L-lysine (Sigma Aldrich, product number: P04832-50ML; 0.01%, incubated at room temperature for 5min, air dried), and Matrigel (Corning, product number: 354234; incubated at 37°C for 30 min). The culture medium used in this experiment was obtained by adding 20 mM BDM, 10% fetal bovine serum, 100 U/ml penicillin and 100 µg/ml streptomycin to M199 medium. The isolated cardiomyocytes were plated on coated 48-well culture plates, cultured for 72 hours (h), and photographed under a microscope. 4 fields of view were taken for each group for calculating the average number of rod-shaped cells, and the result was expressed as the mean ± standard deviation.

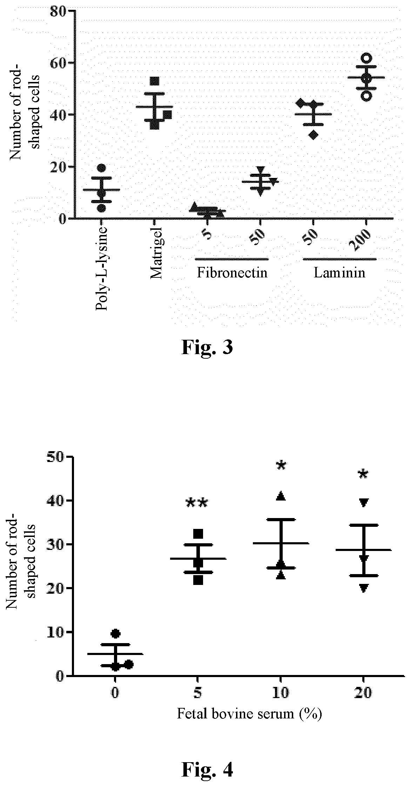

The results were shown in Figure 3. After 72 hours of culture, it was observed that the number of rod-shaped cells in the poly-L-lysine and fibronectin (5 µg/ml, 50 µg/ml) groups was significantly lower than that in the Matrigel group, 50 µg/ml laminin group and 200 µg/ml laminin group, and the 200 µg/ml laminin group had the highest number of rod-shaped cells. This indicated that the use of Matrigel and laminin can allow cardiomyocytes to adhere and grow. The concentration of laminin used as a coating composition in the prior art is usually about 10 µg/ml, and the inventors of the present application have found that when the concentration of laminin is 50-200 µg/ml, cardiomyocytes can effectively adhere to the wall in the present invention.

### Example 2.3: The effect of the serum concentration on the average rate of human rod-shaped cardiomyocytes

20 mM BDM, 100 U/ml penicillin and 100 µg/ml streptomycin were added to M199 medium, and then different concentrations of sera (0%, 5%, 10% and 20% fetal bovine serum) were added respectively. Human cardiomyocytes were plated on a 48-well cell culture plate precoated with 200 µg/ml laminin, and placed and cultured in a 37°C cell incubator (with 5% CO₂, and a relative saturated humidity of 95%) for 5 days.

It can be seen from Figure 4 that the average rate of rod-shaped cells (the proportion of rod-shaped cells in the total cells) in each experimental group was remarkably higher than that in the control group, with a statistical difference (p < 0.05). However, there was no statistical difference among the three experimental groups with different serum concentrations (p > 0.05). The average rate of rod-shaped cells in the 10% fetal bovine serum group was slightly higher than that in the 5% fetal bovine serum group and the 20% fetal bovine serum group. Thus, it can be determined that the suitable fetal bovine serum concentration during the culture of cardiomyocytes was 5%-20%, preferably 10%.

### Example 2.4: The effect of different types of ready-prepared culture media on the survival rate of human cardiomyocytes

The following 8 commonly used ready-prepared culture media were selected, namely MEM (purchased from Thermo), MEM-GlutaMAX (purchased from Thermo), MEM-Gluta MAX-4-hydroxyethyl piperazine ethanesulfonic acid (purchased from Thermo), M199 (purch ased from Sigma), M199 glutamine-free (purchased from Thermo), M199-4-hydroxyethyl pi perazine ethanesulfonic acid glutamine-free (purchased from Thermo), DMEM-high glucose (purchased from Thermo) and DMEM-low glucose-sodium pyruvate (purchased from Ther mo) media.

20 mM BDM, 100 U/ml penicillin and 100 µg/ml streptomycin were added to 8 different ready-prepared culture media respectively. The isolated human cardiomyocytes were plated on a 48-well cell culture plate precoated with 200 µg of laminin, and placed and cultured in a 37°C cell incubator (with 5% CO₂, and a relative saturation humidity of 95%) for 5 days.

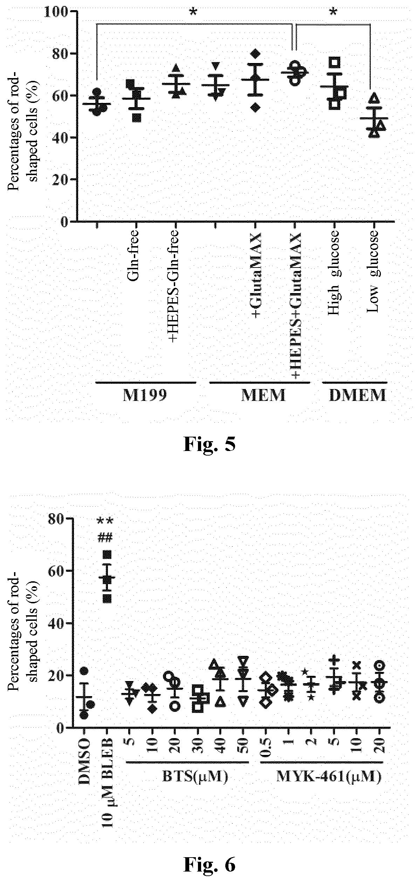

The results were shown in Figure 5. The 8 culture media have reached a rate of rod-shaped cells of about 50%-70% or more, and thus all of them can be used for culturing human cardiomyocytes. In particular, compared with the M199 medium used in Example 2.1, a close, equivalent or even higher rate of rod-shaped cells has been achieved with several other culture media (M199 glutamine-free medium, M199-4-hydroxyethyl piperazine ethanesulfonic acid glutamine-free medium, MEM medium, MEM-GlutaMAX medium, MEM-4-hydroxyethyl piperazine ethanesulfonic acid-GlutaMAX medium, DMEM-high glucose medium, and DMEM-low glucose medium), indicating that the choice of culture medium had little effect on the survival rate of human cardiomyocytes.

### Example 2.5: The effect of myosin inhibitors N-benzyl-p-toluenesulfonamide (BTS) and MYK-461 on the survival rate of human cardiomyocytes

In order to explore whether there are other myosin ATPase inhibitors besides BDM a nd (-)-Blebbistatin that can improve the rate of rod-shaped cardiomyocytes, the effects of (-)-Blebbistatin, BTS, and MYK-461 on the activity of cardiomyocytes were compared.For the isolated human cardiomyocytes, MEM-4-hydroxyethyl piperazine ethanesulfonic acid-Gl utaMAX medium comprising 10 µM (-)-Blebbistatin, BTS (at concentrations of 5 µM, 10 µM, 20 µM, 30 µM, 40 µM, or 50 µM, respectively), or MYK-461 (at concentrations of 0.5 µM, 1 µM, 2 µM, 5 µM, 10 µM, or 20 µM, respectively) respectively was used, and 10% fetal bovine serum, 100 U/ml penicillin and 100 µg/ml streptomycin were adde d to the medium.

The results were shown in Figure 6. the percentages of rod-shaped cells in the BTS and MYK-461 groups at different concentrations were not significantly different, but were significantly lower than the percentage in the (-)-Blebbistatin group (p < 0.05). This further indicated that not all myosin ATPase inhibitors can achieve good results in culturing human cardiomyocytes.

### Example 2.6: The effect of metabolic regulators on the survival rate of human cardiomyocytes

The metabolic regulators sodium pyruvate (2 mM), insulin (10 mg/L), creatine (5 mM), β-aminoethanesulfonic acid (taurine, 5 mM) and L-carnitine (2 mM) were mixed and added to a culture medium, and the mixture of the above-mentioned metabolic regulators was referred to as "PICCT" for short. The experimental groups were divided into three groups by addition of different concentrations of (-)-Blebbistatin (0 µM, 5 µM, and 10 µM) to the basal medium (M199 medium supplemented with 10% fetal bovine serum, 100 U/ml penicillin and 100 µg/ml streptomycin), and each group included two conditions with or without PICCT, to explore the effects of growth factor and metabolic substrates on the survival rate of human cardiomyocytes. After culturing in a 37°C cell incubator (with 5% CO₂, and a relative saturation humidity of 95%) for 7 days, the calibrated survival rate of cardiomyocytes was calculated (cell survival rate on day 7/cell survival rate on day 0 × 100%). During the culture of cardiomyocytes, some cells losed their original long rod-shaped cell morphologies and grew adherently, and these cells were regarded as dead cells and were not counted as living cells.

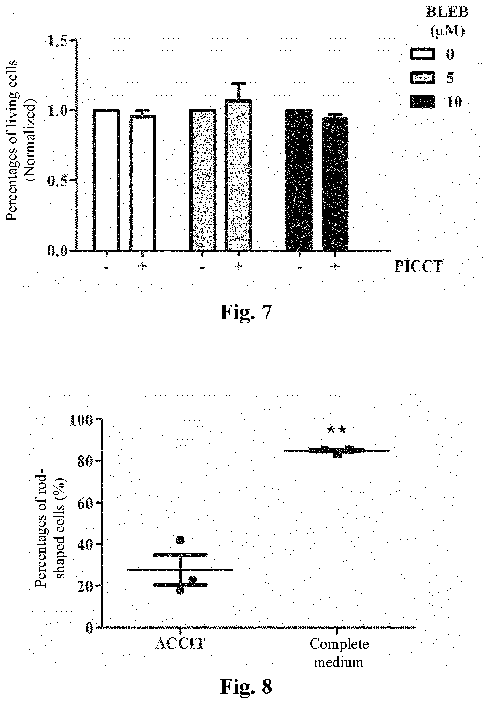

As shown in Figure 7, compared with the (-)-Blebbistatin groups at the corresponding concentrations, the addition of PICCT had no remarkable effect on the survival rate. The above results indicated that the component playing a more critical role in cell survival was (-)-Blebbistatin rather than the metabolic regulator components in PICCT during the cell culture.

### Example 2.7: Comparison of the culture medium of the present invention and ACCIT medium

ACCIT medium recorded in the literature (V. Bistola, M. Nikolopoulou, A. Derventzi, A. Kataki, N. Sfyras, N. Nikou, M. Toutouza, P. Toutouzas, C. Stefanadis, and M. M. Konstadoulakis, "Long-Term Primary Cultures of Human Adult Atrial Cardiac Myocytes: Cell Viability, Structural Properties and Bnp Secretion in Vitro", Int J Cardiol, 131 (2008), 113-22.) (components and contents were shown in Table 5 below) and the culture medium of the present invention (obtained by adding 10 µM (-)-Blebbistatin, 10% fetal bovine serum, 100 U/ml penicillin and 100 µg/ml streptomycin to the MEM-4-hydroxyethyl piperazine ethanesulfonic acid-GlutaMAX ready-prepared medium) were used respectively for culturing human cardiomyocytes for 6 days, and the proportions of rod-shaped cells in total cells were calculated.

The results were shown in Figure 8, the ratio of rod-shaped cardiomyocytes in the complete medium group of the present invention was remarkably higher than that in the ACCIT group, with a statistical difference (p < 0.05). This further indicated that the culture medium of the present invention can achieve an excellent effect of improving the survival rate of human cardiomyocytes.

**[Table 5]**

| Component | Concentration | Component | Concentration |
|---|---|---|---|
| Bovine serum albumin (BSA) | 2 mg/ml | Insulin | 0.1 µM |
| L-carnitine | 2 mM | Penicillin | 100 IU/ml |
| Creatine | 5 mM | Streptomycin | 100 µg/ml |
| β-aminoethanesulfonic acid | 5 mM | | |

In summary, preferred culture conditions for human cardiomyocytes in the present invention were as follows: Matrigel (incubated at 37°C for 30 min) or 50-200 µg/ml laminin (incubated overnight in a 37°C incubator, and air dried) was used to precoat a culture plate, and a culture medium obtained by adding 10%-20% fetal bovine serum, 2.5-20 µM (-)-Blebbistatin, and 100 U/ml penicillin and 100 µg/ml streptomycin to any of ready-prepared media selected from MEM-4-hydroxyethyl piperazine ethanesulfonic acid-GlutaMAX, M199 glutamine-free, M199-4-hydroxyethyl piperazine ethanesulfonic acid glutamine-free, MEM, MEM-GlutaMAX, and DMEM-high glucose media was used.

The above results indicated that the culture medium of the present invention comprising (-)-Blebbistatin can not only significantly improve the survival rate during isolation of human cardiomyocytes, but also can significantly improve the survival rate during the culture of human cardiomyocytes.

### Example 2.8: The effect of culture time on cell morphologies

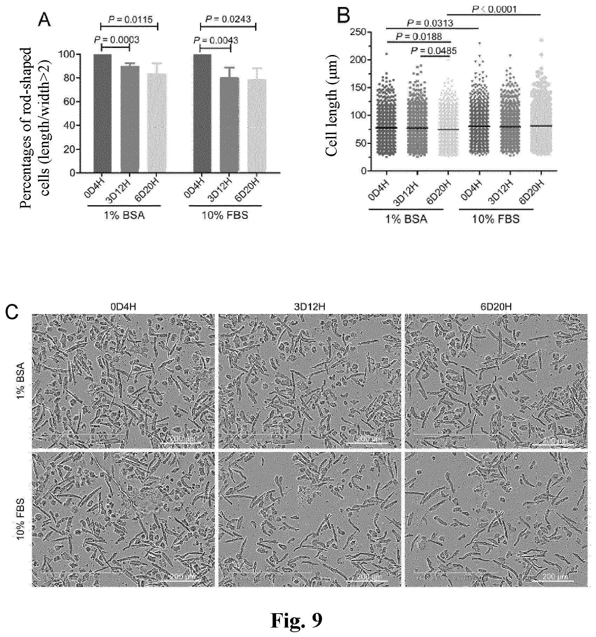

100 µg/ml penicillin, 100 µg/ml streptomycin, 100 µg/ml Primocin and 10 µM (-)Blebbistatin were added to MEM-GlutaMAX to obtain the basal medium used in this experiment. The experiment was divided into 2 groups by addition of BSA and FBS to the basal medium respectively to obtain basal media comprising 1 g/ml BSA and 10% FBS, respectively (note: for simplicity, the concentration unit of BSA in examples and the corresponding figures was sometimes expressed as an approximate mass fraction "%" instead of "g/ml", for example, "1% BSA" means "1 g/ml BSA"). The left atrial appendage tissue was derived from patients aged 45-65 years. The isolated human primary cardiomyocytes were divided into 2 groups by resuspending them in basal media comprising 1% BSA and 10% FBS, respectively. The cells were cultured using a 200 µg/ml laminin-coated 96-well cell culture plate, with 6000 cells seeded per well, and placed and cultured in a 37°C cell incubator (with 5% CO₂, and a relative saturated humidity of 95%) for 7 days. During the culture, the media were changed every two days, and the Incucyte (S3) instrument was used to automatically take pictures every 4 hours. In data processing, the length and width of each cell were measured, and cells with a ratio of length to width greater than 2 were counted as rod-shaped cells. The rate of rod-shaped cells under each culture condition was calculated, and the experiment was repeated 6 times. The results were shown in Figure 9.

Although Figure 9A showed that the rate of rod-shaped cardiomyocytes cultured in the two media decreased with time, Figure 9B showed that the cell length of the 10% FBS group maintained relatively well during the time period tested, and Figure 9C showed that the survival rate of rod-shaped cells in the 1% BSA group was maintained relatively well after the 3rd day.

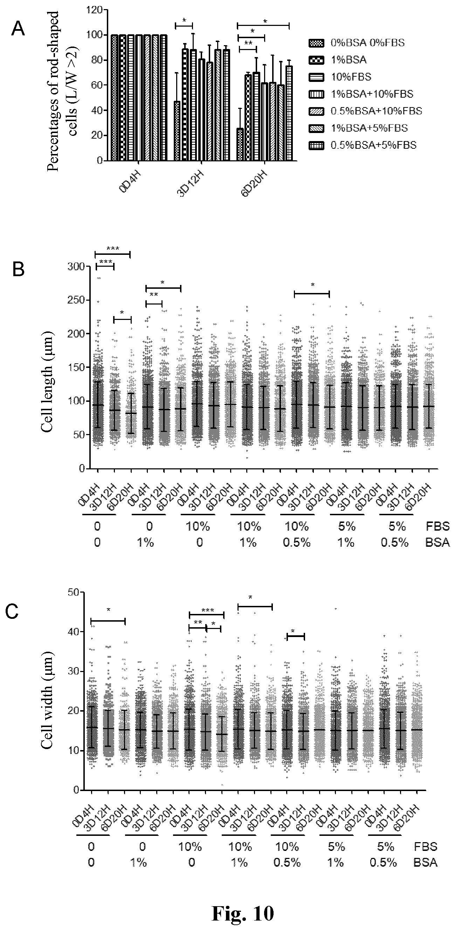

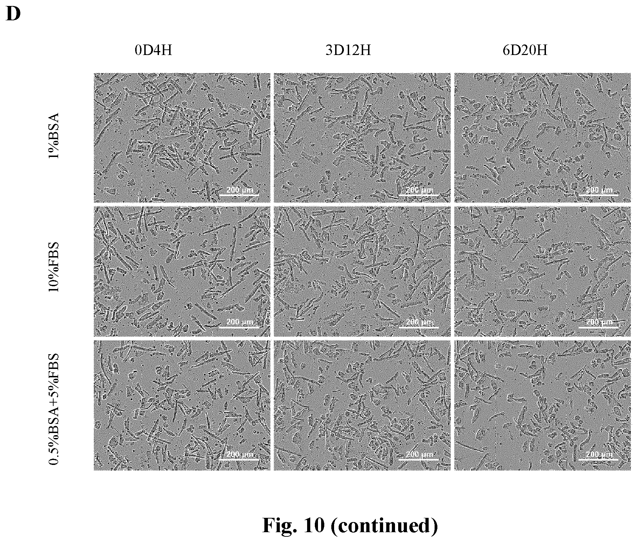

In addition, the present inventors further designed a scheme to use BSA and FBS for cell culture at the same time to detect changes in the rate of rod-shaped cells, cell length, and cell width on days 3 and 6 of culture, in the hope that the simultaneous use of BSA and FBS would reduce the influence on cell morphologies. In particular, there were a total of 7 groups by addition of the following components to the basal medium: 0% BSA+0% FBS, 1% BSA, 10% FBS, 1% BSA+10% FBS, 0.5% BSA+10% FBS, 1% BSA+5% FBS, and 0.5% BSA+5% FBS, and the detection was performed in triplicate. Other experimental conditions remained unchanged. The results were shown in Figure 10, indicating that the cell morphology in each group was maintained at about days 3 and 6.

### Example 2.9: The effect of continuous culture on the level of cell transcription

After isolation, the human primary cardiomyocytes were cultured in the basal media comprising 1% BSA and 10% FBS as described in Example 2.8, and the levels of cell transcription one day and three days after culture were compared with those on the day of isolation. In particular, the cell samples thus obtained were centrifuged first respectively, and then the cell pellets from which the supernatant slurries were discarded were stored in a refrigerator at -80°C. RNAs were extracted with GeneJET RNA Purification Kit (Thermo Scientific, K0731), and the RNA concentration and mass were determined with NanoDrop 2000 (Thermo Scientific). Then KAPA mRNA HyperPrep Kit (Illumina, KK8581) was used to construct a cDNA library with 500 ng of RNAs. The size and concentration of the cDNA library were determined using 2100 Bioanalyzer (Agilent) and Qubit (Thermo Fisher Scientific) respectively. The library was diluted to 2-3 pM, and sequenced using the Illumina NextSeq 500 sequencing platform with the corresponding kit being 75 cycles of NextSeq 500/550 High Output Kit v2.5 kit (Illumina, 20024906).

According to the sequencing results, a heat map was generated, the number of up-regulated and down-regulated genes was counted and the change amounts (|log2FoldChange|) of up-regulated and down-regulated genes were determined. Log2FoldChange ≥ 0.58 indicated up-regulation, and log2FoldChange ≤ -0.58 indicated down-regulation. Changes of D1 up-regulation, D1 down-regulation, D3 up-regulation, and D3 down-regulation in 1% BSA and 10% FBS groups were compared using a t-test.

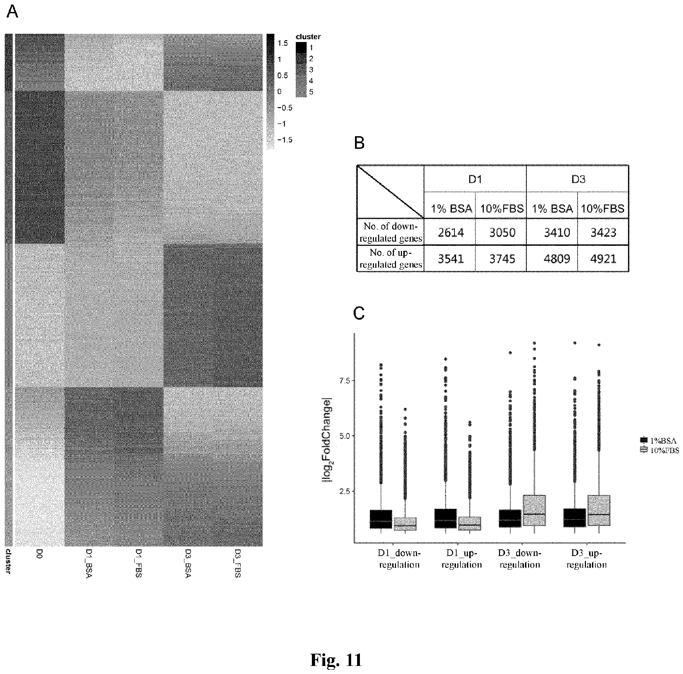

It can be seen from Figure 11A that the 1% BSA and 10% FBS groups had basically the same trend of gene up-regulation and down-regulation changes on days 1 and 3 of culture, indicating that both groups can maintain the original cell state of cardiomyocytes. Figure 11B showed that the number of up-regulated and down-regulated genes on days 1 and 3 of culture suggested that the number of genes changed in the 1% BSA group was even less than that in the 10% FBS group; and Figure 11C showed that in the up-regulated genes, the degrees of change in the 1% BSA group on days 1 and 3 of culture were also less than in the 10% FBS group, indicating that the effect of the 1% BSA group was even better than that of the 10% FBS group.

## Claims

1. A composition comprising (-)-Blebbistatin and/or para-aminoblebbistatin, and optionally one or more components selected from energy components, metabolic regulators, acidifying or alkalizing agents, antibiotics and isotonic agents, wherein
(-)-Blebbistatin is a compound represented by the following Formula (1), 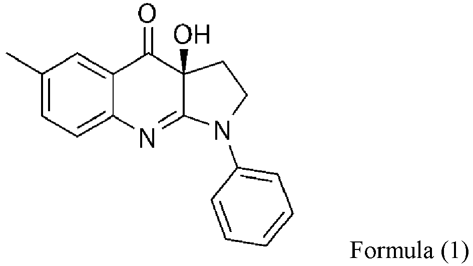
para-aminoblebbistatin is a compound represented by the following Formula (2),

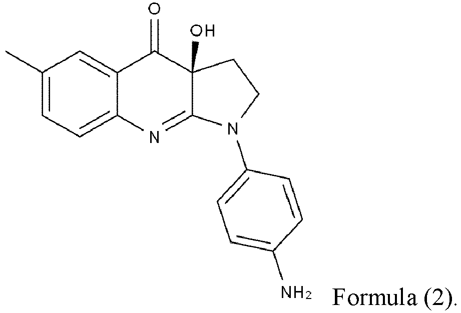

2. The composition of claim 1, comprising the following components: 1-50 µM (-)-Blebbistatin, 5-50 mM glucose, 0.1-20 mM sodium pyruvate, 0.1-20 mM creatine, 5-50 mM β-aminoethanesulfonic acid, 0.5-10 mM 4-hydroxyethyl piperazine ethanesulfonic acid (HEPES), 10-500 U/ml penicillin, 10-500 µg/ml streptomycin, 0.1-20 mM magnesium chloride, 0.1-20 mM potassium chloride, 10-500 mM sodium chloride, 0.5-30 mM sodium dihydrogen phosphate, and an appropriate amount of NaOH to make the pH of the composition be 7.0-7.8.

3. A method for isolating cardiomyocytes, comprising the steps of calcium-free perfusion, digestion and cell collection, wherein the composition of claim 1 or 2 is used in the steps of calcium-free perfusion, digestion and cell collection.

4. A composition obtained by adding (-)-Blebbistatin and/or para-aminoblebbistatin, antibiotics and sera to M199 medium series, MEM medium series, and DMEM medium series.

5. The composition of claim 4, which is obtained by adding the following components to M199 medium, MEM-GlutaMAX medium or MEM-4-hydroxyethyl piperazine ethanesulfonic acid-GlutaMAX medium:
• (-)-Blebbistatin and/or para-aminoblebbistatin, wherein in the composition, the concentration of (-)-Blebbistatin is 1-50 µM, and the concentration of para-aminoblebbistatin is 1-100 µM;
• penicillin, and/or streptomycin, and/or Primocin, wherein the concentration of penicillin in the composition is 10-500 U/ml, the concentration of streptomycin in the composition is 10-500 µg/ml, and the concentration of Primocin in the composition is 10-500 µg/ml; and
• fetal bovine serum and/or bovine serum albumin, wherein the volume fraction of the fetal bovine serum in the composition is 1%-20%, and the concentration of the bovine serum albumin in the composition is 0.1-10 g/ml.

6. A kit, comprising:
(1) the composition of claim 4 or 5; and
(2) a coating composition, comprising Matrigel or laminin.

7. A method for culturing cardiomyocytes, wherein the composition of claim 4 or 5 is used as a culture medium.

8. The method for culturing cardiomyocytes of claim 7, wherein a culture plate is coated with the coating composition before culturing the cells.

9. The kit of claim 6 or the method of claim 8, wherein the coating composition comprises Matrigel and/or 10-500 µg/ml laminin.

10. Use of (-)-Blebbistatin and/or para-aminoblebbistatin in the preparation of a cardiomyocyte isolation reagent or a cardiomyocyte culture reagent.
